# EUROPÄISCHE PATENTSCHRIFT

(11) **EP 2 763 628 B1**
(45) Veröffentlichungstag und Bekanntmachung des Hinweises auf die Patenterteilung: **04.03.2015**
(21) Anmeldenummer: 12775617.9
(22) Anmeldetag: 02.10.2012
(51) Int. Cl.: A61F 2/72, A61B 5/0488, A61F 2/54, A61F 2/60

(54) **PROTHESENEINRICHTUNG**
PROSTHESIS ASSEMBLY
APPAREIL PROTHÉTIQUE

(30) Priorität: 06.10.2011 DE 102011114920
(43) Veröffentlichungstag der Anmeldung: 13.08.2014
(73) Patentinhaber: Otto Bock HealthCare GmbH, 37115 Duderstadt (DE)
(72) Erfinder: FÖRSTER, Heiko, 37115 Duderstadt (DE); HEUBLEIN, Christian, 37115 Duderstadt (DE)
(74) Vertreter: Stornebel, Kai
(86) Internationale Anmeldenummer: PCT/EP2012/004125
(87) Internationale Veröffentlichungsnummer: WO 2013/050135

(56) Entgegenhaltungen:
- EP-B1- 1 411 872
- DE-A1-102005 021 412
- DE-A1-102008 036 714
- DE-A1-102009 030 217
- DE-U1-202006 007 460
- GB-A- 1 191 633
- US-A- 5 413 611
- US-A1- 2009 216 339

## Beschreibung

Die Erfindung betrifft eine Protheseneinrichtung mit einem Außenschaft, der eine proximale Öffnung und an seinem distalen Ende Anschlussmittel zum Befestigen einer zusätzlichen Komponente aufweist und aus einem in Längserstreckung starren Material ausgebildet ist, in dem zumindest ein zumindest teilweise in Längserstreckung verlaufender Schlitz eingebracht ist, um eine Verformung in Radialrichtung zu ermöglichen, einem Innenschaft, der aus einem flexiblen Material ausgebildet ist und eine proximale Öffnung aufweist, und zumindest einer Spanneinrichtung, die an dem Außenschaft festgelegt und über die eine Durchmesserveränderung des Außenschaftes erreichbar ist. Eine solche Protheseneinrichtung ist insbesondere für sogenannte Testprothesen oder Übergangsprothesen vorteilhaft, die nicht durch Abformen des Amputationsstumpfes individuell angepasst werden.

Prothesen sind seit langer Zeit bekannt und dienen als Ersatz für nicht vorhandene, beispielsweise verlorene Gliedmaßen. Funktionselemente, beispielsweise Prothesenhände oder Prothesenfüße, werden dabei an einem formstabilen Außenschaft befestigt, der den Amputationsstumpf allseitig umgibt und in der Regel trichterförmig ausgebildet ist. Der Außenschaft dient dazu, die Kraft von dem Amputationsstumpf auf das Funktionselement zu übertragen. Außenschäfte werden aus faserteilverstärkten Kunststoffen, Holz oder Metall hergestellt und werden regelmäßig möglichst exakt an den Amputationsstumpf angepasst, um neben einem hohen Tragekomfort mit einer optimalen Abstützwirkung unter anderem einen Unterdruck erzeugen zu können, um den Prothesenschaft am Amputationsstumpf halten zu können.

Problematisch ist die Tatsache, dass das Volumen eines Amputationsstumpfes über die Zeit variiert, beispielsweise schwillt ein Amputationsstumpf nach der Amputation oder einer Operation zunächst an und dann wieder ab. Ziel einer Prothesenversorgung ist es jedoch, den Patienten möglichst schnell an die prothetische Versorgung zu gewöhnen. Um innerhalb vorgegebener Grenzen eine Anpassung an Amputationsstümpfe zu ermöglichen, deren Durchmesser und Länge sich verändern, ist in der EP 1 411 872 B1 eine Prothese beschrieben, die einen Silikonliner mit Kupplungsstift, einen mit Längsschlitzen versehen und mittels Spannelementen im Durchmesser veränderbaren Prothesenschaft sowie einen Halter zum Verbinden einer künstlichen Gliedmaße mit dem Prothesenschaft beschrieben, bei dem die Längsschlitze überbrückt sind. Der Prothesenschaft besitzt einen konzentrischen Bund, in dem ein zylindrischer Adapter höhenverstellbar befestigt ist.

Die US 2009/216339 A1 betrifft Prothesen, genauer gesagt ein System zum Bereitstellen myoelektrischer Signale durch Liner zum Betreiben myoelektrisch gesteuerter, angetriebener Prothesen und allgemein das Bereitstellen myoelektrischer Signale durch nicht leitende Barrieren. Ein System aus einem Liner und einer Elektrode sieht einen flexiblen, leitenden, gewebebasierten Elektrodeneinsatz auf der Innenseite eines Liners vor, einen zweiten Abschnitt, der durch den Liner hindurchgeht und einen dritten Abschnitt auf der äußeren Oberfläche des Liners, wobei alle drei Teile einstückig ausgebildet sind, also die Elektrode den Liner durchragt.

Aufgabe der vorliegenden Erfindung ist es, eine Protheseneinrichtung bereitzustellen, mit der schnell und einfach an unterschiedlichen Patienten eine erste Versorgung auch mit angetriebenen Komponenten oder Funktionselementen, die an dem Außenschaft befestigt sind und mit myoelektrischen Signalen gesteuert werden, durchgeführt werden kann.

Erfindungsgemäß wird diese Aufgabe durch eine Protheseneinrichtung mit den Merkmalen des Hauptanspruches gelöst, vorteilhafte Ausgestaltungen und Weiterbildungen der Erfindung sind in den Unteransprüchen, der Beschreibung und den Figuren offenbart.

Die erfindungsgemäße Protheseneinrichtung mit einem Außenschaft, der eine proximale Öffnung und an seinem distalen Ende Anschlussmittel zum Befestigen einer zusätzlichen Komponente aufweist und aus einem in Längserstreckung starren Material ausgebildet ist, in dem zumindest ein zumindest teilweise in Längserstreckung verlaufender Schlitz eingebracht ist, um eine Verformung in Radialrichtung zur ermöglichen, einem Innenschaft, der aus einem flexiblen, ggf. elastischen und dehnbaren Material ausgebildet ist und eine proximale Öffnung aufweist, und zumindest einer Spanneinrichtung, die an dem Außenschaft festgelegt und über die eine Durchmesserveränderung des Außenschaftes erreichbar ist, sieht vor, dass auf einer dem Innenschaft zugewandten Seite des Außenschaftes zumindest eine Elektrode verlagerbar befestigt ist.

Durch die verlagerbare Befestigung einer Elektrode auf der Innenseite des Außenschaftes ist es möglich, eine schnelle und einfache Testmöglichkeit myoelektrischer Versorgungen an einem Patienten durchzuführen, ohne dass auf aufwendige Art und Weise ein Abdruck des Amputationsstumpfes genommen und eine Elektrode aufwendig in dem Außenschaft installiert werden muss. Auf diese Weise kann auch ohne Bereitstellung einer individuellen Protheseneinrichtung eine Testversorgung durchgeführt werden, so dass beispielsweise geeignete Prothesenkomponenten wie angetriebene Prothesenhände oder Prothesenkniegelenke vor der endgültigen Anpassung des Außenschaftes auf eine grundsätzliche Geeignetheit überprüft werden können. Auf diese Weise können hohe Kosten bei der Patientenversorgung vermieden werden, da bereits im Vorfeld überprüft werden kann, ob und welche angetriebene Prothesenkomponente, die an dem Außenschaft befestigt werden kann, geeignet ist, wie Elektroden anzuordnen sind und wo eine vorteilhafte Anordnung der Elektroden zur Steuerung der Komponenten erfolgen kann.

Eine Weiterbildung der Erfindung sieht vor, dass an dem Innenschaft zumindest ein Areal mit leitfähigen Elementen vorgesehen ist, die myoelektrische Signale von der Innenseite des Innenschaftes zu der Außenseite des Innenschaftes leiten. Durch das Vorsehen zumindest eines Areals mit leitfähigen Elementen oder aus einem leitfähigen Material, das myoelektrische Signale von der Hautoberfläche des Amputationsstumpfes an die Außenseite des Innenschaftes leitet, ist es möglich, einen relativ großen Hautoberflächenbereich abzudecken, der zum Ableiten myoelektrischer Signale geeignet ist. Es ist nicht mehr notwendig, Oberflächenelektroden aufwendig auf der Hautoberfläche zu positionieren, zu fixieren und dann per Kabel die Signale an eine Steuerungseinrichtung weiterzuleiten, vielmehr kann durch eine ungefähre Positionierung des Areals mit leitfähigen Elementen ohne großen Aufwand ein großes Gebiet möglicher myoelektrischer Signalquellen abgedeckt werden, dem dann die an dem Außenschaft angeordnete Elektrode zugeordnet wird. Die Zuordnung der Elektrode zu dem Areal mit leitfähigen Elementen oder aus leitfähigem Material folgt dann nach Maßgabe der Art des Signals und der Qualität des Signals, also welchem Muskel das Signal zugeordnet werden kann oder welche Signalstärke vorhanden ist.

Die leitfähigen Elemente können in dem Innenschaft eingebettet oder das Areal aus Bereichen aus einem leitfähigen Kunststoff ausgebildet sein. Grundsätzlich besteht auch die Möglichkeit, dass der gesamte Innenschaft aus einem leitfähigen Material ausgebildet ist, bei dem Bereiche durch Isolierabschnitte elektrisch voneinander getrennt sind, so dass entlang einer sehr großen Oberfläche myoelektrische Signale abgenommen werden können. Dadurch ist es möglich, eine schnelle und einfache Adaption myoelektrischer Elektroden vorzunehmen, da die am Außenschaft angeordnete Elektrode oder Elektroden leicht veränderbar positioniert werden kann oder können. Die Veränderung der Positionierung kann auch noch bei angelegter Protheseneinrichtung erfolgen, beispielsweise, wenn die Spanneinrichtung gelöst ist, so dass der Außenschaft radial nach außen aufgebogen werden kann.

Der Innenschaft kann an dem Außenschaft fixiert sein, vorteilhafterweise findet eine permanente Fixierung des Innenschaftes an dem Außenschaft statt, so dass die Protheseneinrichtung als System aus Innenschaft, Außenschaft und Spanneinrichtung zusammen mit der gegebenenfalls ebenfalls permanent aber verlagerbar an dem Außenschaft angeordneten Elektrode gehandhabt werden kann. Die Anordnung des Innenschaftes an dem Außenschaft erfolgt vorteilhafterweise so, dass bei einem begrenzten Areal mit leitfähigen Elementen oder aus einem leitfähigen Material dieses so angeordnet ist, dass die Möglichkeit des Ableitens myoelektrischer Signale gegeben ist und dass eine räumliche Zuordnung der Elektrode zu dem Areal gegeben ist.

Der Innenschaft weist vorteilhafterweise einen geschlossenen Querschnitt auf, so dass er aufgrund des dehnbaren und elastischen Materials leicht an den Amputationsstumpf angelegt werden kann. Der Innenschaft ist damit als variabler, anpassbarer und dennoch festsitzender Innenschaft ausgebildet, der die Schnittstelle zu dem Amputationsstumpf bildet. Die maximale Dehnbarkeit des Innenschaftes ist vorteilhafterweise an die radiale Flexibilität des Außenschaftes angepasst, so dass gegebenenfalls vorgefertigte Größen in unterschiedlichen Durchmesserabstufungen vorgesehen sein können. So können für verschiedene Patiententypen Protheseneinrichtungen in unterschiedlichen Größen vorbereitet werden, die den gesamten oder nahezu den gesamten Größenbereich der zu versorgenden Patienten abdecken.

Der Innenschaft kann ein offenes, distales Ende aufweisen, so dass aufgrund der Öffnung einerseits kein Druck auf das distale Stumpfende ausgeübt wird und andererseits eine Längenanpassbarkeit durch eine Verlagerung des Innenschaftes auf dem Amputationsstumpf auch in proximaler Richtung möglich ist.

Der Innenschaft ist vorteilhafterweise aus Silikon ausgebildet und kann bereichsweise aus einem leitfähigen Silikon oder einem Gemisch aus einem Silikon und einem anderen Kunststoff oder leitfähigen Material bestehen. Der Außenschaft besteht vorteilhafterweise vollständig aus einem faserverstärkten Kunststoff, um bei einem möglichst geringen

Die Elektrode zwischen dem Innenschaft und dem Außenschaft kann in zumindest einer Führung, die an dem Außenschaft angeordnet ausgebildet ist, verschieblich und/oder verdrehbar gelagert sein. Ebenfalls ist es möglich, die Elektrode durch eine besondere Oberflächenausgestaltung durch Formschlusselement, beispielsweise Klettverschluss, an der Innenseite des Außenschaftes zu befestigen.

Die Elektrode ist vorteilhafterweise in Längserstreckung und in Umfangsrichtung des Außenschaftes verlagerbar gelagert, so dass die Elektrode oder die Elektroden relativ frei an dem Außenschaft positioniert werden kann bzw. können.

Sperreinrichtungen können als Band oder Riemen ausgebildet sein und die maximale Durchmesserveränderung des Außenschaftes festlegen. Dazu ist das Band oder der Riemen geschlossen ausgebildet bzw. so an dem Außenschaft festgelegt, dass ein Entfernen oder Trennen des Bandes oder des Riemens nicht möglich ist, so dass der Außenschaft und damit auch der Innenschaft nicht über einen vorgesehenen Grenzwert hin aufgebogen werden können. Durch die Spanneinrichtung wird auch die maximale Durchmesserveränderung bei einer Kompression festgelegt. Die Spanneinrichtung kann einen Ratschenverschluss, einen Hebelverschluss, einen Drehspannverschluss und/oder einen Klettverschluss aufweisen, beispielsweise kann der Ratschenverschluss als ein Drehverschluss ausgebildet sein, der durch Drehen in eine Richtung eine Spannwirkung hervorruft und durch eine andere Aktuierung ein Lösen ermöglicht.

Das Material des Innenschaftes kann elastisch und dehnbar ausgebildet sein, alternativ dazu kann der Innenschaft einen Schlitz in Längserstreckung aufweisen. Eine seitliche Öffnung in dem Innenschaft kann eine Umfangsanpassung ermöglichen. Überlappende Abschnitte, die im Überlappungsbereich ggf. eine verringerte Materialstärke aufweisen, so dass eine im Wesentlichen gleichbleibende Materialstärke beibehalten werden kann, erfüllt ebenfalls die Anforderungen, die an eine Umfangsanpassbarkeit gestellt werden. Es können zu diesem Zweck auch elastische und dehnbare Einsätze in dem Innenschaft eingebracht sein.

Der Schlitz in dem Außenschaft kann teilweise oder vollständig mit einem elastischen Material, z. B. Elastomer, ausgefüllt sein, um beispielsweise ein Einklemmen des Innenschafts zu vermeiden, ohne die Aufweitbarkeit zu beeinträchtigen. Ebenso kann durch das Elastomer der Verformungswiderstand beeinflusst werden.

Nachfolgend wird ein Ausführungsbeispiel der Erfindung anhand der beigefügten Figuren näher erläutert. Es zeigen:
- Figur 1 -: eine Rückansicht einer Protheseneinrichtung;
- Figur 2 -: eine Vorderansicht einer Protheseneinrichtung;
- Figur 3 -: eine Seitenansicht einer Protheseneinrichtung;
- Figur 4 -: eine Seitenansicht eines Innenschaftes;
- Figur 5 -: eine Vorderansicht eines Innenschaftes;
- Figur 6 -: Ansichten einer Elektrode;
- Figur 7 -: eine Seitenansicht einer Variante des Innenschaftes; sowie
- Figur 8 -: eine Vorderansicht der Variante gemäß Figur 7.

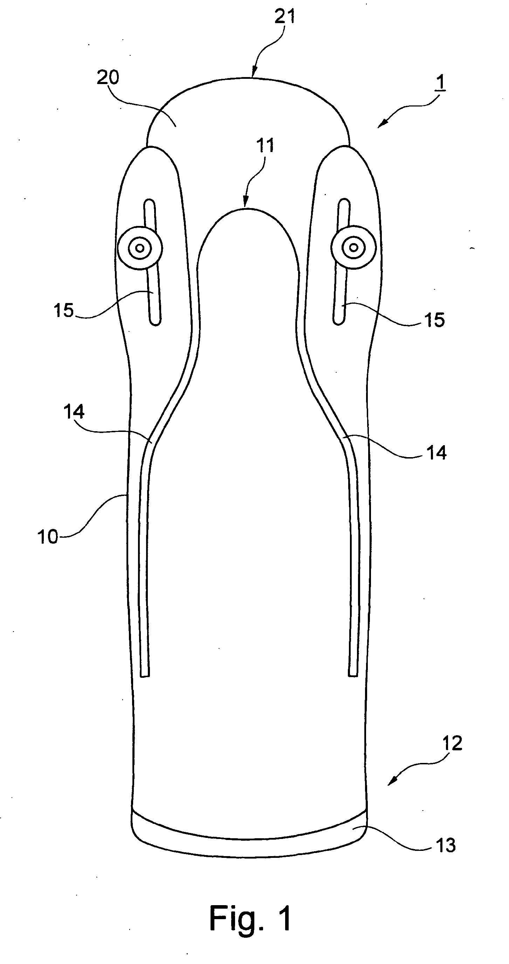

In der Figur 1 ist in einer Rückansicht eine Protheseneinrichtung 1 in Gestalt einer Unterarmprothese dargestellt. Die Protheseneinrichtung 1 weist einen Außenschaft 10 und einen darin angeordneten und daran befestigten Innenschaft 20 auf. Die Protheseneinrichtung 1 wird gemäß dem dargestellten Ausführungsbeispiel an einem Unterarmstumpf getragen, der Unterarmstumpf ist nicht gezeigt. Alternativ wird Anlegen der Protheseneinrichtung 1 der Unterarmstumpf in den Innenschaft 20 durch eine proximale Öffnung 21 eingeführt und anschließend der Innenschaft 20 mit dem Unterarmstumpf in eine proximale Öffnung 11 des Außenschaftes 10 eingeführt und daran festgelegt.

Der Außenschaft 10 besteht aus einem strukturfesten Material, vorzugsweise einem faserverstärkten Kunststoff, bei dem Fasermatten, beispielsweise Glasfasermatten oder Kohlefasermatten, in einer Kunststoffmatrix eingebettet sind. Durch dieses Kompositmaterial ist es möglich, bei geringen Wandstärken eine hohe Festigkeit bei gleichzeitig geringem Gewicht zu erreichen. In der Längserstreckung des Außenschaftes 10 werden durch dieses Material sehr starre Eigenschaften verwirklicht, entlang der Längserstreckung ist ein Stauchen oder ein Verlängern oder Ausdehnen des Außenschaftes 10 nicht oder nur in einem extrem geringen Maße möglich. Der Außenschaft 10 ist im Wesentlichen zylindrisch aufgebaut und entspricht in seiner Kontur im Wesentlichen der Kontur eines Unterarmes. An dem distalen Ende 12 des Außenschaftes 10 sind Anschlussmittel 13 für weitere Komponenten vorgesehen, beispielsweise für eine Prothesenhand oder dergleichen. Über die Anschlussmittel 13 kann eine feste Kopplung der zusätzlichen Komponenten mit der Protheseneinrichtung 1 erfolgen. Die Anschlussmittel 13 erlauben eine reversible Befestigung der zusätzlichen Komponente, um eine Anpassung an den jeweiligen Nutzungszweck zu ermöglichen.

Innerhalb des Außenrahmens 10 sind Schlitze 14 eingearbeitet, die im Wesentlichen in Längserstreckung der Protheseneinrichtung 1 angeordnet sind. Die Schlitze 14 verlaufen im Wesentlichen klappsymmetrisch zu einer nicht eingezeichneten Klappachse, die in Längserstreckung des Außenschaftes 10 verläuft, abweichende Verläufe sind möglich und vorgesehen. Die Schlitze 14 enden vor dem distalen Ende 12 des Außenschaftes 10, so dass sich am distalen Ende 12 ein im Wesentlicher geschlossener Querschnitt des Außenschaftes 10 in einer Rohrform ergibt. Durch die Schlitze 14 ist eine Beweglichkeit des Außenschaftes 10 in Radialrichtung möglich. Die durch die Schlitze 14 ausgebildeten Segmente des Außenschaftes 10, die am distalen Ende 12 miteinander verbunden sind, können sich somit radial nach innen und außen verlagern, wobei der Außenschaft 10 in Radialrichtung vorzugsweise elastisch ausgebildet ist, so dass ausgehend von einer Ausgangsstellung bei einem Aufbiegen oder Zusammendrücken der Segmente eine Widerstandskraft überwunden werden muss.

Weiterhin sind an dem Außenschaft 10 Führungen 15 in Gestalt von Schlitzen vorgesehen, die im dargestellten Ausführungsbeispiel ebenfalls im Wesentlichen in Längserstreckung des Außenschaftes 10 orientiert angeordnet sind. Die Führungen 15 können auch als offener Schlitz ausgebildet sein, also nicht vollständig von dem Material des Außenschaftes 10 umgeben sein, ebenso ist es möglich, dass die Orientierung der Führungen 15 in Umfangsrichtung verläuft oder eine Kurvenbahn beschreibt.

Der Innenschaft 20 mit der proximalen Öffnung 21 kann fest an dem Außenschaft 10 befestigt sein, beispielsweise über Nieten, Schrauben, Formschlusselemente oder dergleichen, alternativ dazu kann durch eine Formgebung des Außenschaftes 10 ein formschlüssiges Verriegeln des Innenschaftes 20 mit dem Außenschaft 10 erfolgen. Der Innenschaft 20 ist vorzugsweise aus Silikon ausgebildet, alternative Materialien können vorgesehen sein. Die Länge des Innenschaftes 20 hängt von der Länge des Außenschaftes 10 sowie von der Länge des zu versorgenden Stumpfes ab. Üblicherweise endet der Innenschaft 20 vor dem distalen Ende 12 des Außenschaftes 10. Der Innenschaft 20 weist vorteilhafterweise einen geschlossenen Querschnitt auf, er kann aber auch ein offenes distales Ende aufweisen, so dass Längenschwankungen des zu versorgenden Stumpfes ausgeglichen werden können und darüber hinaus kein Druck auf das gegebenenfalls noch empfindliche distale Ende des Stumpfes ausgeübt wird. Das Material des Innenschaftes 20 ist vorteilhafterweise elastisch und dehnbar ausgebildet, ebenfalls ist es möglich, dass ein Schlitz in Längserstreckung des Innenschaftes 20 vorhanden ist, so dass der Innenschaft 20 zumindest abschnittsweise aus zwei einander überlappenden Lagen besteht. Diese Lagen können im Verhältnis zu dem übrigen Material des Innenschaftes 20 dünner ausgebildet sein, so dass sich im Bereich der Überdeckung keine Materialaufdickung ergibt.

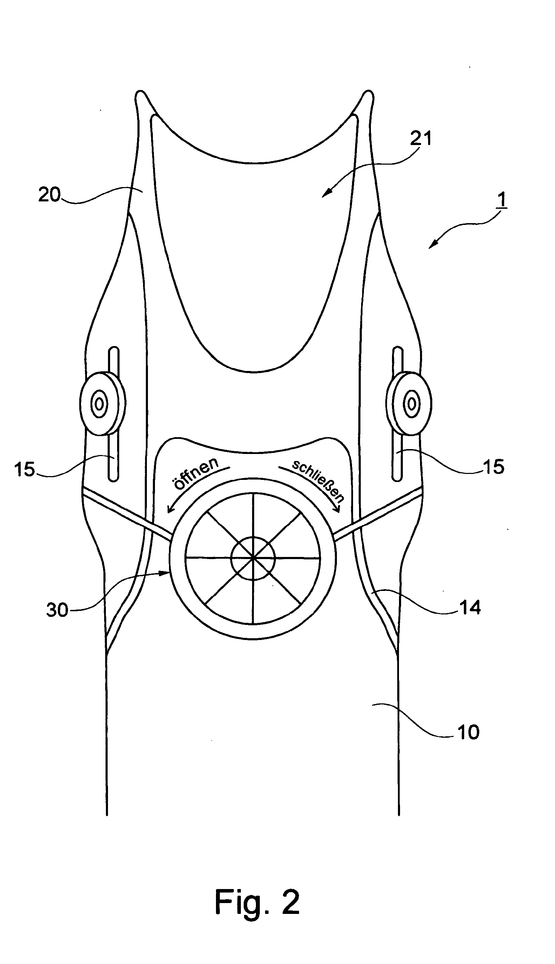

In der Figur 2 ist die Protheseneinrichtung 1 in Vorderansicht dargestellt. Der Innenschaft 20 mit der proximalen Öffnung 21 ist ebenso zu erkennen wie der Außenschaft 10 mit den in Längserstreckung verlaufenden Schlitzen 14 und den Führungen 15, die medial und lateral angeordnet sind. Ergänzend zu der Figur 1 ist in der Figur 2 eine Spanneinrichtung 30 in Gestalt eines Drehspannverschlusses dargestellt, über den eine Durchmesserveränderung des Außenschaftes erreichbar ist. Durch Drehen in die ein oder andere Richtung ist ein Schließen oder Öffnen der Spanneinrichtung 30 durch ein Verlängern und ein Verkürzen der der Spanneinrichtung 30 zugeordneten Bänder oder Kabel möglich. Aufgrund der Schlitze 14, die sich in Längserstreckung des Außenschaftes 10 erstrecken, ergeben sich im proximalen Bereich des Außenschaftes 10 vier voneinander getrennte Segmente, die medial, lateral und dorsal und ventral orientiert sind. In der Figur 2 ist zu erkennen, dass der Drehspannverschluss 30 auf der ventral angeordneten Seite des Außenschaftes 10 angeordnet ist, also auf dem Segment, das der Ellenbeuge zugewandt ist.

Der Innenschaft 20 kann aus einem Kunststoff oder Silikon bestehen, der bereichsweise unterschiedliche Shore-Härten aufweist, so dass der darin aufzunehmende Stumpf optimal eingebettet wird.

Über den Drehspannverschluss 30 ist es möglich, eine Umfangsveränderung des Spannmittels zu erreichen, so dass eine radial wirkende Kraft auf den Außenschaft 10, den Innenschaft 20 und darüber auf den Stumpf aufgebracht wird. Dadurch ist es möglich, die Protheseneinrichtung 1 über die Spanneinrichtung 30 individuell auf den Stumpf des Nutzers der Protheseneinrichtung 1 einzustellen und an ihn anzupassen. Durch diese Einrichtung ist es möglich, einen vorgefertigten Außenschaft 10 oder mehrere Außenschäfte in Standardgrößenabstufungen vorrätig zu haben und diese dann individuell an den Patienten anzupassen, so dass die teure und aufwendige individuelle Anpassung durch Abnahme eines Gipsmodells und Anfertigung eines Prothesenschaftes mit faserverstärkten Verbundmaterialien nicht notwendig wird. Eine solche Protheseneinrichtung kann bevorzugt als eine sogenannte Testprothese eingesetzt werden, so dass die grundsätzliche Eignung einer solchen Protheseneinrichtung für einen Patienten ohne großen finanziellen Aufwand überprüft werden kann. Insbesondere ist eine solche Testprotheseneinrichtung für Patienten sinnvoll, die erstmalig mit einer angetrieben Protheseneinrichtung ausgestattet werden, die durch myoelektrische Signale gesteuert wird.

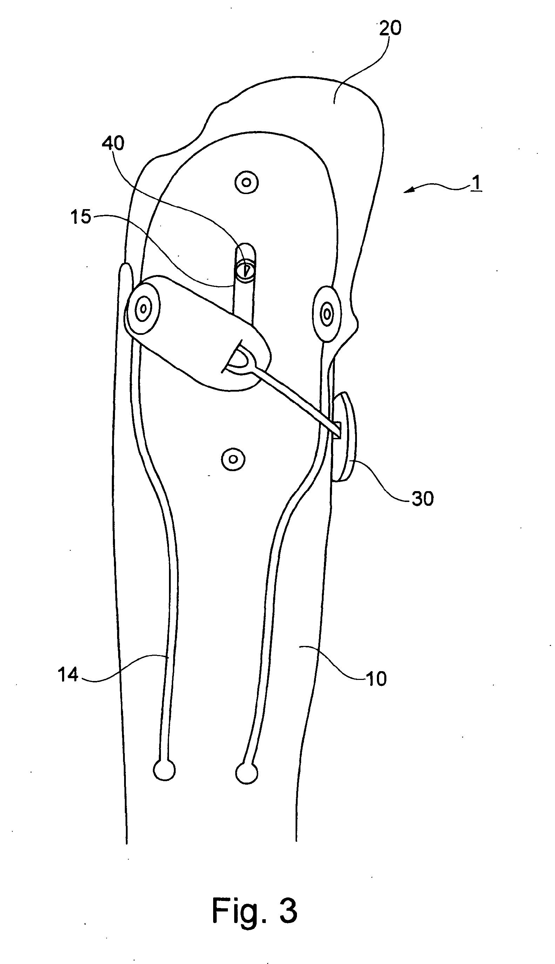

In der Figur 3 ist in einer Seitenansicht die Protheseneinrichtung 1 mit dem Außenschaft 10, dem Innenschaft 20 und der Spanneinrichtung 30 dargestellt. In der Figur 3 ist zu erkennen, dass in den Führungen 15 der medial und lateral orientierten Segmente eine Elektrode 40 verlagerbar geführt ist. Ein Bolzen oder Zapfen ist an der Elektrode 40 befestigt und ragt durch die als Schlitz ausgebildete Führung 15 zur Außenseite des Außenschaftes 10, so dass die Elektrode 40 zwischen der Innenseite des Außenschaftes 10 und der Außenseite des Innenschaftes 20 angeordnet ist. Entlang der Führung 15 kann die Elektrode 40 bewegt werden, vorliegend entlang im Wesentlichen der Längserstreckung des Außenschaftes 10. Innerhalb der Führung 15 kann die Elektrode 40 verdrehbar gelagert sein, so dass eine optimale Orientierung der Elektrode 40 an dem Patienten vorgenommen werden kann. Über Feststelleinrichtungen, beispielsweise Schraubgewinde, Keile, Clipse oder dergleichen kann die für günstig erachtete Position der Elektrode 40 an dem Außenschaft 10 fixiert werden.

In der Figur 3 ist weiterhin zu erkennen, dass das Spannmittel 30 im Wesentlichen umfänglich um den Außenschaft 40 herum geführt ist. So können zwei Gurte von dem Drehspannverschluss 30 umfänglich um den Außenschaft herumgeführt und am gegenüberliegenden Segment, hier dem dorsalen Endsegment, festgelegt werden. Durch Drehen in Uhrzeigerrichtung wird die Spannung auf das Zugmittel erhöht, so das eine Durchmesserveränderung des Außenschaftes 10 durch eine Verlagerung der durch die Schlitze 14 getrennten Segmente aufeinander zu erfolgen kann. Entgegen der Uhrzeigerrichtung wird der Drehspannverschluss 30 betätigt, federt der Außenschaft 10 in die Ausgangsposition zurück. Sperreinrichtungen können vorgesehen sein, um ein übergroßes Aufweiten des Außenschaftes 10 und des Innenschaftes 20 zu vermeiden. Dazu kann die Spanneinrichtung 30 mit einem Endanschlag ausgestattet sein, so dass bei Erreichen einer Maximalaufweitung eine Sperrwirkung eintritt, alternativ ist eine separate Sperreinrichtung möglich.

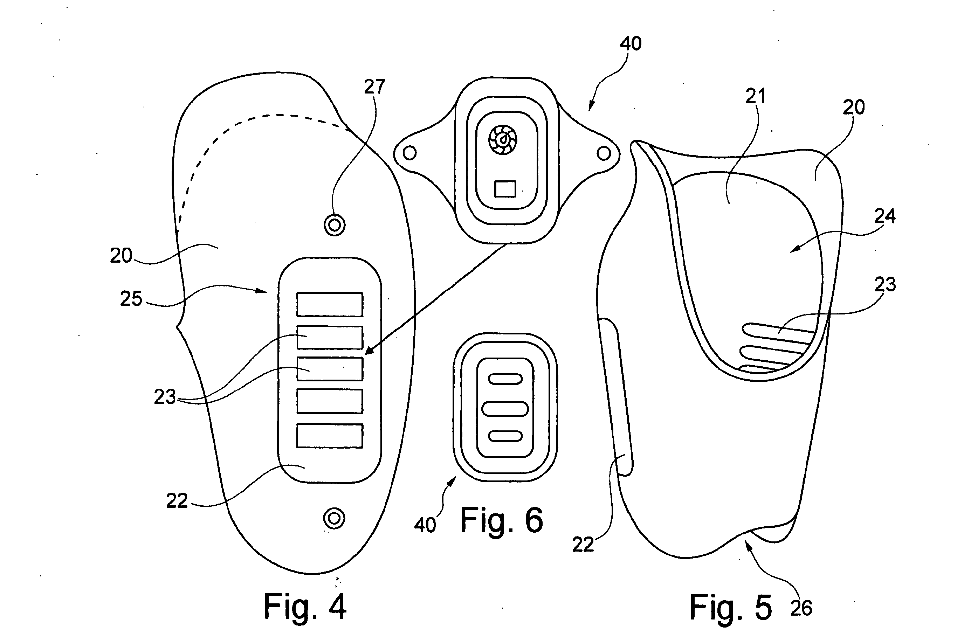

In der Figur 4 ist der Innenschaft 20 in einer Seitenansicht dargestellt. Der Innenschaft 20 weist ein Areal 22 an der medialen Seite des Innenschaftes 20 auf, in dem eine Vielzahl leitfähiger Elemente 23 angeordnet sind. In dem dargestellten Ausführungsbeispiel sind die leitfähigen Elemente 23 als parallele, im Wesentlichen rechteckige Elemente 23 ausgebildet, mit denen myoelektrische Signale von der Hautoberfläche von einer Innenseite des Innenschaftes 20 zu einer Außenseite 25 des Innenschaftes 20 geleitet werden können. Ebenfalls sind Befestigungselemente 26 an dem Innenschaft vorgesehen, mit denen der Innenschaft 20 an dem Außenschaft 10 festlegbar ist.

In der Figur 5 ist eine Vorderansicht des Innenschaftes 20 mit der proximalen Öffnung 21 und der distalen Öffnung 26 zu erkennen. Der Figur 5 ist zu entnehmen, dass die leitfähigen Elemente 23 auch an einer Innenseite 24 des Innenschaftes 20 oberflächlich durchragen, so dass myoelektrische Signale von dem nicht dargestellten Stumpf von der Innenseite 24 des Innenschaftes 20 durch die leitfähigen Elemente 23 zur Außenseite 25 des Innenschaftes 20 geleitet werden können. Der Figur 5 ist ebenfalls zu entnehmen, dass zwei Areale 22 mit leitfähigen Elementen 23 an dem Innenschaft 20 vorgesehen sind, nämlich medial und lateral angeordnet. Grundsätzlich ist es auch möglich, mehr als zwei Areale 22 mit leitfähigen Elementen 23 an dem Innenschaft 20 anzuordnen.

In der Figur 6 sind zwei Ansichten einer Elektrode 40 dargestellt. Die obere Darstellung zeigt die Außenseite der Elektrode 40, die untere Darstellung die Innenseite der Elektrode 40. Im eingebauten Zustand, wie er in der Figur 3 gezeigt ist, ist die Elektrode 40 mit der Innenseite dem Areal 22 mit den leitfähigen Elementen 23 zugeordnet. Auf der Innenseite der Elektrode 40 sind Sensoren oder Aufnehmer zu erkennen, die korrespondierend zu den leitfähigen Elementen 23 ausgebildet sind. Durch Verschieben der montierten Elektrode 40 an dem Außenschaft 10 innerhalb der Führung 15 ist es möglich, eine vorläufige optimale Position der Elektrode 40 zum Erhalt eines oder mehrerer myoelektrischer Signale einzustellen. Dazu ist es nicht notwendig, Oberflächenelektroden auf der Haut des Patienten festzulegen und bereits eine Zuordnung der Position der Elektrode zu einem Bereich der Hautoberfläche herzustellen. Vielmehr ist es möglich, durch die Vielzahl der leitfähigen Elemente 23 einen großen Bereich potentieller Ableitstellen für myoelektrische Signale abzudecken, der durch die Position der Elektrode 40 auf der Außenseite 25 des Innenschaftes 20 in dem Areal 22 der leitfähigen Elemente 23 festgelegt wird, wobei die Position der Elektrode 40 innerhalb des Areals 22 im Bereich dessen, was die Führung 15 zulässt, frei wählbar ist.

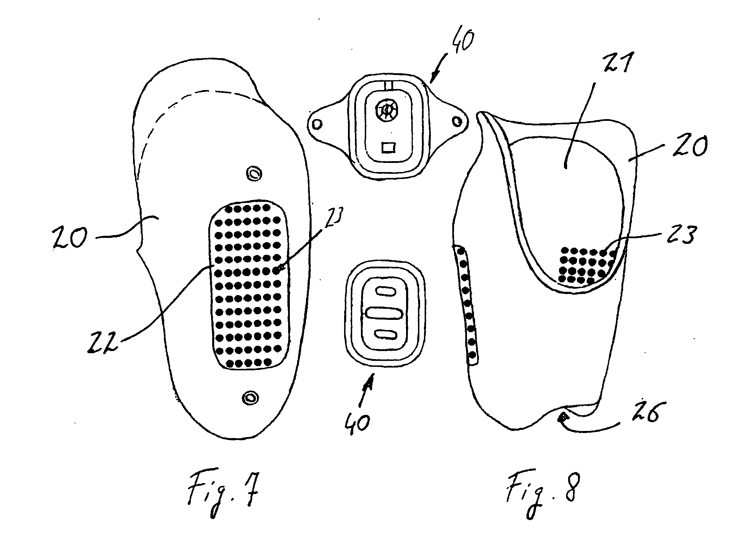

In den Figuren 7 und 8 ist eine Variante der Erfindung dargestellt, bei der auf dem Innenschaft 20 statt der eckigen leitfähigen Elemente runde leitfähige Elemente 23 in den Arealen 22 angeordnet sind. Die elektrisch leitenden Elemente 23 können nachträglich in den Innenschaft 20 eingesetzt sein, ebenfalls ist es möglich, dass der Innenschaft 20 aus einem leitfähigen Material besteht, das durch Isoliermaterial elektrisch getrennt wird. Ebenfalls ist es möglich, dass in den Arealen 22 leitfähiges Silikon oder leitfähiger Kunststoff als leitfähige Elemente 23 in elektrisch voneinander entkoppelten bzw. isolierten Bereichen angeordnet sind, um die leitfähigen Elemente 23 an unterschiedlichen Stellen des Areals 22 auszubilden.

## Patentansprüche

1. Protheseneinrichtung mit
• einem Außenschaft (10), der eine proximale Öffnung (11) und an seinem distalen Ende (12) Anschlussmittel (13) zum Befestigen einer zusätzlichen Komponente aufweist und aus einem in Längserstreckung starren Material ausgebildet ist, in dem zumindest ein zumindest teilweise in Längserstreckung verlaufender Schlitz (14) eingebracht ist, um eine Verformung in Radialrichtung zu ermöglichen,
• einem Innenschaft (20), der aus einem flexiblen Material ausgebildet ist und eine proximale Öffnung (21) aufweist,
• zumindest einer Spanneinrichtung (30), die an dem Außenschaft (10) festgelegt und über die eine Durchmesserveränderung des Außenschaftes (10) erreichbar ist,
**dadurch gekennzeichnet, dass** auf einer dem Innenschaft (20) zugewandten Seite des Außenschaftes (10) zumindest eine Elektrode (40) verlagerbar befestigt ist.

2. Protheseneinrichtung nach Anspruch 1, **dadurch gekennzeichnet, dass** an dem Innenschaft (20) zumindest ein Areal (22) mit leitfähigen Elementen (23) vorgesehen ist, die myoelektrische Signale von der Innenseite (24) des Innenschaftes (20) zu der Außenseite (25) des Innenschaftes (20) leiten.

3. Protheseneinrichtung nach Anspruch 2, **dadurch gekennzeichnet, dass** die leitfähigen Elemente (23) in dem Innenschaft (20) eingebettet oder das Areal (22) als leitfähiger Kunststoff ausgebildet ist.

4. Protheseneinrichtung nach einem der voranstehenden Ansprüche, **dadurch gekennzeichnet, dass** der Innenschaft (20) an dem Außenschaft (10) fixiert ist.

5. Protheseneinrichtung nach einem der voranstehenden Ansprüche, **dadurch gekennzeichnet, dass** der Innenschaft (20) einen geschlossenen Querschnitt aufweist.

6. Protheseneinrichtung nach einem der voranstehenden Ansprüche, **dadurch gekennzeichnet, dass** der Innenschaft (20) ein offenes distales Ende (26) aufweist.

7. Protheseneinrichtung nach einem der voranstehenden Ansprüche, **dadurch gekennzeichnet, dass** die Elektrode (40) in zumindest einer Führung (15), die an dem Außenschaft (10) angeordnet oder ausgebildet ist, verschieblich und/oder verdrehbar gelagert ist.

8. Protheseneinrichtung nach einem der voranstehenden Ansprüche, **dadurch gekennzeichnet, dass** die Spanneinrichtung (30) als Band oder Riemen ausgebildet ist und die maximale Durchmesserveränderung des Außenschaftes (10) festlegt.

9. Protheseneinrichtung nach einem der voranstehenden Ansprüche, **dadurch gekennzeichnet, dass** die Spanneinrichtung (30) einen Ratschenverschluss, Hebelverschluss, Drehspannverschluss oder Klettverschluss aufweist.

10. Protheseneinrichtung nach einem der voranstehenden Ansprüchen, **dadurch gekennzeichnet, dass** das Material des Innenschafes (20) elastisch und dehnbar ausgebildet ist oder einen Schlitz in Längserstreckung des Innenschaftes (20) aufweist.

## Claims

1. A prosthetic assembly, comprising:
• an outer socket (10), which has a proximal opening (11) and, at the distal end (12) thereof, connection means (13) for fastening an additional component, and is formed from a material rigid in the direction of longitudinal extent, wherein at least one slit (14) extending at least partially in the direction of longitudinal extent is introduced into said outer socket in order to enable a deformation in the radial direction,
• an inner socket (20), which is formed from a flexible material and has a proximal opening (21),
• at least one tensioning appliance (30), which is fixed on the outer socket (10) and by means of which a change in diameter of the outer socket (10) is achievable, **characterized in that** at least one electrode (40) is displaceably fastened on a side of the outer socket (10) facing the inner socket (20).

2. The prosthetic assembly as claimed in claim 1, **characterized in that** at least one area (22) with conductive elements (23) is provided on the inner socket (20), which conductive elements conduct myoelectric signals from the inner side (24) of the inner socket (20) to the outer side (25) of the inner socket (20).

3. The prosthetic assembly as claimed in claim 2, **characterized in that** the conductive elements (23) are embedded in the inner socket (20) or the area (22) is embodied as a conductive plastic.

4. The prosthetic assembly as claimed in one of the preceding claims, **characterized in that** the inner socket (20) is affixed to the outer socket (10).

5. The prosthetic assembly as claimed in one of the preceding claims, **characterized in that** the inner socket (20) has a closed cross section.

6. The prosthetic assembly as claimed in one of the preceding claims, **characterized in that** the inner socket (20) has an open distal end (26).

7. The prosthetic assembly as claimed in one of the preceding claims, **characterized in that** the electrode (40) is displaceably and/or rotatably mounted in at least one guide (15) which is arranged or formed on the outer socket (10).

8. The prosthetic assembly as claimed in one of the preceding claims, **characterized in that** the tensioning appliance (30) is embodied as tape or belt and sets the maximum change in diameter of the outer socket (10).

9. The prosthetic assembly as claimed in one of the preceding claims, **characterized in that** the tensioning appliance (30) has a ratchet lock, a lever lock, a rotary clamping lock or a hook-and-loop lock.

10. The prosthetic assembly as claimed in one of the preceding claims, **characterized in that** the material of the inner socket (20) is embodied to be elastic and stretchable or has a slit in the direction of longitudinal extent of the inner socket (20).

## Revendications

1. Système formant prothèse comprenant
- une tige extérieure (10), qui comporte une ouverture proximale (11) et des moyens de raccordement (13) à son extrémité distale (12) pour la fixation de composants additionnels, et qui est réalisée à partir d'un matériau rigide dans son extension longitudinale, dans lequel est ménagée au moins une fente (14) s'étendant au moins partiellement dans l'extension longitudinale, afin de permettre une déformation en direction radiale,
- une tige intérieure (20), qui est réalisée en un matériau flexible et qui comporte une ouverture proximale (20),
- au moins un moyen de tensionnement (30), qui est fixé sur la tige extérieure (10) et au moyen duquel peut être obtenue une modification de diamètre de la tige extérieure (10),
**caractérisé en ce qu'**au moins une électrode (40) est fixée, de façon déplaçable, sur un côté de la tige extérieure (10) tourné vers la tige intérieure (20).

2. Système formant prothèse selon la revendication 1, **caractérisé en ce qu'**il est prévu sur la tige intérieure (20) au moins une zone (22) avec des éléments conducteurs (23), qui mène des signaux myoélectriques depuis le côté intérieur (24) de la tige intérieure (30) vers la face extérieure (25) de la tige intérieure (20).

3. Système formant prothèse selon la revendication 2, **caractérisé en ce que** les éléments conducteurs (23) sont noyés dans la tige intérieure (20), ou bien **en ce que** la zone (22) est réalisée sous la forme d'une matière plastique conductrice.

4. Système formant prothèse selon l'une des revendications précédentes, **caractérisé en ce que** la tige intérieure (20) est fixée sur la tige extérieure (10).

5. Système formant prothèse selon l'une des revendications précédentes, **caractérisé en ce que** la tige intérieure (20) présente une section transversale fermée.

6. Système formant prothèse selon l'une des revendications précédentes, **caractérisé en ce que** la tige intérieure (20) comporte une extrémité distale ouverte (26).

7. Système formant prothèse selon l'une des revendications précédentes, **caractérisé en ce que** l'électrode (40) est montée d'une manière capable de translation et/ou de rotation dans au moins un guidage (15) qui est agencé ou réalisé sur la tige extérieure (10).

8. Système formant prothèse selon l'une des revendications précédentes, **caractérisé en ce que** le dispositif de tensionnement (30) est formé comme une bande ou une courroie et fixe la modification maximale du diamètre de la tige extérieure (10).

9. Système formant prothèse selon l'une des revendications précédentes, **caractérisé en ce que** le dispositif de tensionnement (20) comprend une fermeture à cliquet, une fermeture à levier, une fermeture à serrage par rotation ou une fermeture agrippante.

10. Système formant prothèse selon l'une des revendications précédentes, **caractérisé en ce que** le matériau de la tige intérieure (20) est réalisé de manière élastique et capable de s'allonger, ou comporte une fente dans l'extension longitudinale de la tige intérieure (20).
